# EUROPEAN PATENT APPLICATION

(11) **EP 2 630 925 A1**
(43) Date of publication of application: **28.08.2013**
(21) Application number: 11844225.0
(22) Date of filing: 21.11.2011
(51) Int. Cl.: A61B 17/28, A61B 19/00

(54) **TREATMENT TOOL FOR MEDICAL USE, AND MANIPULATOR**

(30) Priority: 30.11.2010 JP 2010267775
(71) Applicant: Olympus Corporation, Shibuya-ku Tokyo 151-0072 (JP)
(72) Inventor: HYODO Ryoji, Tokyo 151-0072 (JP)
(74) Representative: Schicker, Silvia
(86) International application number: PCT/JP2011/076782
(87) International publication number: WO 2012/073738

(57) **Abstract**

This medical treatment tool (1) includes: a treatment portion (10) having a first forceps piece (11) and a second forceps piece (12) that are coupled to each other so as to be relatively rotatable about a forceps rotating shaft (13); an operation member (20) at least a part of which has flexibility and a distal end of which is connected to a proximal end of the treatment portion (10); and a sheath portion (30) which has flexibility and through which the operation member (20) is inserted so as to be capable of advancing and retracting in a longitudinal direction of the sheath portion, wherein in the operation member (20), a range with a predetermined length from a distal end is a hard portion (20A) with rigidity higher than that of an other portion, a connecting portion between the treatment portion (10) and the operation member (20) is set so as to be located outside the sheath portion (30) in a state with the operation member (20) being retracted as much as possible, and the length of the hard portion (20A) is set so that the other portion is not exposed to an outside of the sheath portion (30) in a state with the operation member (20) being advanced as much as possible.

## Description

### Technical Field of the Invention

The present invention relates to a medical treatment tool and a manipulator provided with the same. Priority is claimed **o**n Japanese Patent Application No. 2010-267775, filed on November 30, 2010, the content of which is incorporated herein by reference.

### Background Art

Conventionally, in the field of medical practice, medical treatment tools for grasping living tissues, surgical tools, or the like are used when manipulations are performed. These medical treatment tools are introduced into the body cavities of the patients and the like after, for example, being attached to a manipulator employed in a master-slave-type medical manipulator system or being inserted through a channel for a pair of forceps of an endoscope. Then, the medical treatment tools are used for a variety of manipulations.

Patent Literature 1 describes, as one of the medical treatment tools, a pair of straight grasping forceps provided with openable forceps portion. To the forceps portion, a wire is connected via a link mechanism. The wire is inserted through a coil sheath. When the wire is pushed/pulled to be advanced/retracted in the longitudinal direction, the forceps portion opens/closes.

### Citation List

### Patent Literature

Patent Literature 1: Japanese Examined Utility Model Application, Second Publication No. H06-1696

### Summary of the Invention

### Problems to be Solved by the Invention

In the grasping forceps described in Patent Literature 1, a connecting portion between the link mechanism and the wire is located inside the sheath in a state where the wire is retracted as much as possible. Since the radial size of the connecting portion is larger than that of the wire, in this configuration, it is necessary to select a coil sheath in which the connecting portion can be placed, and therefore it is difficult to reduce the diameter of the treatment tool.

The purpose of the present invention is to provide a medical treatment tool whose diameter is easily reduced.
In addition, the other purpose of the present invention is to provide a manipulator a part of which, which performs a manipulation inside a body, is reduced in size.

### Means for Solving the Problem

A medical treatment tool as a first aspect of the present invention includes: a treatment portion having a pair of forceps pieces that are coupled to each other so as to be relatively rotatable about a forceps rotating shaft; an operation member at least a part of which has flexibility and a distal end of which is connected to a proximal end of the treatment portion; and a sheath portion which has flexibility and through which the operation member is inserted so as to be capable of advancing and retracting in a longitudinal direction of the sheath portion, wherein in the operation member, a range with a predetermined length from a distal end is a hard portion with rigidity higher than that of the other portion, wherein a connecting portion between the treatment portion and the operation member is set so as to be located outside the sheath portion in a state with the operation member being retracted as much as possible, and wherein a length of the hard portion is set so that the other portion is not exposed to an outside of the sheath portion in a state with the operation member being advanced as much as possible.

It is preferable that the hard portion is formed by subjecting a surface of the operation member to coating.

It is preferable that a manipulator as a second aspect of the present invention includes the medical treatment tool according to the first aspect of the present invention.
It is preferable that the manipulator further includes a treatment portion rotating mechanism that rotates the treatment portion about a treatment portion rotating axis, and that in a state with the sheath portion being in a straight line and the operation member being retracted as much as possible, a boundary between the hard portion and the other portion is closer to the treatment portion than the treatment portion rotating axis.

### Effects of the Invention

According to the medical treatment tool of the present invention, the diameter of the medical treatment tool can be easily reduced.
In addition, according to the manipulator of the present invention, a part of the medical manipulator which performs a manipulation inside a body can be easily reduced in size, and thereby the manipulation can be favorably performed.

### Brief Description of the Drawings

FIG. 1 is a diagram showing an exemplary construction of a medical manipulator system in which a medical treatment tool of the present invention is used.
FIG. 2 is a diagram showing a distal end of the medical treatment tool.
FIG. 3 is a cross-sectional view showing an internal portion of the medical treatment tool.
FIG. 4 is a diagram showing a motion of the medical treatment tool when in use.
FIG. 5 is a diagram showing a state in which a treatment portion is operated to swing to its limit.
FIG. 6 is a diagram showing a member of an operation member in a modification of the medical treatment tool.
FIG. 7 is a diagram showing a member of an operation member in a modification of the medical treatment tool.
FIG. 8 is a diagram showing a member of an operation member in a modification of the medical treatment tool.

### Embodiments of the Invention

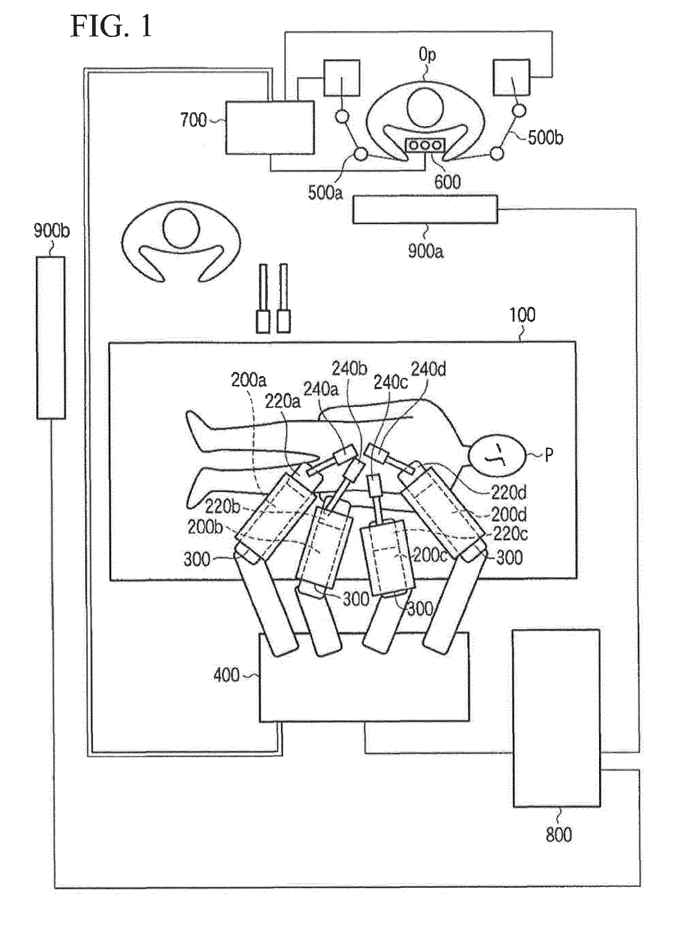

A first embodiment of the present invention will be described with reference to FIG. 1 to FIG. 8. Firstly, an exemplary medical manipulator system in which a medical treatment tool of the present embodiment (hereinafter, referred to simply as "treatment tool") and a manipulator is used will be described.

FIG. 1 is a diagram showing an exemplary medical manipulator system, which is a master-slave-type medical manipulator system. A master-slave-type medical manipulator system refers to a system with two types of arms made of a master arm and a slave arm in which the slave arm is remotely controlled so as to follow the motion of the master arm. The manipulator of the present embodiment can be used as this slave arm.

The medical manipulator system shown in FIG. 1 includes: a surgical table 100; slave arms (manipulators) 200a to 200d; a slave control circuit 400; master arms 500a, 500b; an operation portion 600; an input processing circuit 700; an image processing circuit 800; and displays 900a, 900b.

The surgical table 100 is a table on which a patient P as a target of observation/treatment is to be mounted. In the vicinity of the surgical table 100, the slave arms 200a, 200b, 200c, and 200d are installed. Note that the slave arms 200a to 200d may be installed on the surgical table 100.

Each of the slave arms 200a, 200b, 200c, and 200d has a plurality of multi-degree-of-freedom joints. Each of the slave arms folds their multi-degree-of-freedom joints, to thereby position the treatment tools or the like attached to the distal end sides of the slave arms 200a to 200d (the side in the directed toward the body cavity of the patient P) with respect to the patient P mounted on the surgical table 100. The multi-degree-of-freedom joints are driven individually by a motive power unit not shown in the figure. As the motive power unit, for example a motor (servo motor) provided with an incremental encoder, a decelerator, or the like may be used. The motions of the slave arms are controlled by the slave control circuit 400.
The slave arms 200a to 200d also have their respective motive power units (not shown in the figure) for driving treatment tools 240a to 240d attached thereto. As these motive power units, for example servo motors may also be used. The motions thereof are controlled by the slave control circuit 400.

When the motive power units of the slave arms 200a to 200d driven, the driving amounts of the motive power units are detected by a position detector. Detection signals from the position detector are input to the slave control circuit 400. With the detection signals, the driving amounts of the slave arms 200a to 200d are detected by the slave control circuit 400.

Adaptors for transmitting motive power for surgery (hereinafter, each referred to simply as "adaptor") 220a, 220b, 220c, and 220d are interposed respectively between the slave arms 200a, 200b, 200c, 200d and the treatment tools 240a, 240b, 240c, 240d to connect the slave arms 200a, 200b, 200c, 200d respectively to the treatment tools 240a, 240b, 240c, 240d. Each of the adaptors 220a to 220d has a linear-motion mechanism, and is constructed so as to transmit the motive power generated in the motive power unit of the corresponding slave arm to the corresponding treatment tool through the linear motion.

The slave control circuit 400 includes, for example, a CPU, memory, and the like. The slave control circuit 400 stores a predetermined program for controlling the slave arms 200a to 200d, and controls the motions of the slave arms 200a to 200d or the treatment tools 240a to 240d based on the control signals from the input processing circuit 700. Namely, based on the control signal from the input processing circuit 700, the slave control circuit 400 set a slave arm (or a treatment tool) as an operation target of the master arm operated by an operator Op. Then, the slave control circuit 400 calculates a driving amount necessary for causing the determined slave arm or the like to move correspondingly to the operation amount of the master arm by the operator Op.

In accordance with the calculated driving amount, the slave control circuit 400 controls the slave arm or the like as an operation target of the master arm. At this time, the slave control circuit 400 inputs a driving signal to the corresponding slave arm. Furthermore, in accordance with the detection signal that is input from the position detector of the motive power unit in accordance with the motion of the corresponding slave arm, the slave control circuit 400 controls the intensity and polarity of the driving signal so that the driving amount of the slave arm as an operation target is equal to the target driving amount.

Each of the master arms 500a, 500b is made of a plurality of link mechanisms. Each of the links that constitute the link mechanism is provided with a position detector such as an incremental encoder. With the detection of the motions of the links by the position detectors, the operation amounts of the master arms 500a, 500b are detected by the input processing circuit 700.

In the medical manipulator system of FIG. 1, the two master arms 500a, 500b are used to operate the four slave arm. Therefore, there arises a necessity of appropriately switching the slave arms as operation targets of the master arms. Such switching is performed through, for example, an operation on an operation portion 600 by the operator Op. Obviously, such switching is unnecessary if the master arms and the slave arms are the same in number, namely, the master arms have operation targets on a one-on-one basis.

The operation portion 600 has a variety of operation members such as: a switching button for switching the slave arms as the operation targets of the master arms 500a, 500b; a scaling modification switch for modifying the ratio of the motion between the master and the slave; and a foot switch for shutting down the system in emergency. In the case when any of the operation members constituting the operation portion 600 is operated by the operator Op, the operation signal in accordance with the operation of the corresponding operation member is input from the operation portion 600 to the input processing circuit 700.

The input processing circuit 700 analyzes the operation signals from the master arms 500a, 500b and the operation signal from the operation portion 600. In accordance with the analysis results of the operation signals, the input processing circuit 700 generates a control signal for controlling the medical manipulator system, and input it to the slave control circuit 400.

The image processing circuit 800 performs a variety of image processing operations for displaying the image signal, which has been input from the slave control circuit 400, to thereby produce image data for display on an operator display 900a and on an assistant display 900b. For the operator display 900a and the assistant display 900b, for example liquid crystal displays are used. In accordance with the image data obtained via an observation tool, the displays display an image based on the image data produced by the image processing circuit 800.

In the medical manipulator system constructed as described above, when the operator Op operates the master arms 500a, 500b, the corresponding slave arms and the treatment tools attached to the slave arms move correspondingly to the movements of the master arms 500a, 500b. As a result, it is possible to perform a desired manipulation on the patient P.

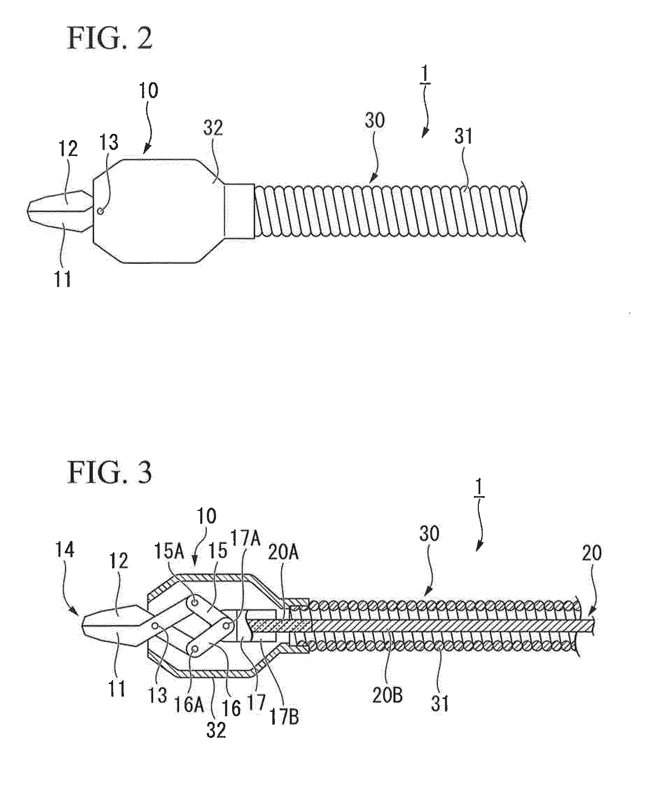

Next, the treatment tool 1 of the present embodiment will be described. FIG 2 shows a distal end of the treatment tool 1. FIG. 3 is a cross-sectional view showing an internal portion of the treatment tool 1. The treatment tool 1 is attachable to the slave arms 200a to 200d as the aforementioned treatment tools 240a to 240d. As shown in FIG. 2 and FIG. 3, the treatment tool 1 includes: a treatment portion 10 for performing a variety of treatments; an operation member 20 for operating the treatment portion 10; and a sheath portion 30 through which the operation member 20 is inserted.

The treatment portion 10 includes a pair of forceps pieces made of a first forceps piece 11 and a second forceps piece 12. The first forceps piece 11 and the second forceps piece 12 are rotatably coupled to each other by a forceps rotating shaft 13. The regions on the side farther forward than the forceps rotating shaft 13 function as a gripping portion 14 that opens and closes to grip a target such as a body tissue, a surgical tool, and the like.

To the proximal end side (the side opposite to the gripping portion 14) of the first forceps piece 11, a link member 15 is rotatably coupled to the first forceps piece 11 via a link rotating shaft 15A. Similarly, to the proximal end side of the second forceps piece 12, a link member 16 is rotatably coupled to the second forceps piece 12 via a link rotating shaft 16A. The shaft lines of the link rotating shafts 15A, 16A are both parallel to the shaft line of the forceps rotating shaft 13.

The proximal ends of the link members 15 and 16 are rotatably connected to a connecting member 17 via a connecting rotating shaft 17A. The shaft line of the connecting rotating shaft 17A is parallel to the forceps rotating shaft 13 and to the link rotating shafts 15A, 16A. The link members 15, 16 are relatively rotatable with respect to the connecting member 17.
The connecting member 17 is made of metal or the like. The connecting member 17 has the connecting rotating shaft 17A at its distal end. The proximal end of the connecting member 17 is an operation member connecting portion 17B that is formed in a substantially cylindrical shape. The distal end of the operation member 20 is inserted into the operation member connecting portion 17B. They are integrally connected to each other by welding, bonding, swaging, or the like.

The operation member 20 is a linear member. The operation member 20 is a member that has both of rigidity to a degree capable of transmitting the operation of advancing/retracting at the proximal end to the distal end and flexibility to a degree capable of bending by following the swing motion of the treatment portion 10, which will be described later. The operation member 20 of the present embodiment is a metal wire made of metal strands being twisted. For example, nine strands made of stainless steel with a diameter of 0.07 millimeters (mm) are twisted to form a wire with an outer diameter of approximately 0.3 mm.
The distal end of the operation member 20 is subjected to coating for increasing its rigidity. As a result, the distal end of the operation member is a hard portion 20A with increased rigidity. As a material for coating for forming the hard portion 20A, a comparatively hard, metal material or the like is preferable.

The hard portion 20A is inserted into the operation member connecting portion 17B of the connecting member 17. This integrally connects the operation member 20 to the connecting member 17 at the proximal end of the treatment portion 10. The size of the hard portion 20A in the axis line direction of the operation member 20 is set to a predetermined length. With the operation member 20 and the connecting member 17 being integrally connected, a part of the hard portion 20A is present outside the operation member connecting portion 17B. The details of the predetermined length will be described later.

The sheath portion 30 includes a cylinder-shaped sheath 31. Through the sheath 31, the operation member 20 is inserted so as to be capable of advancing and retracting. In the present embodiment, a known coil sheath with flexibility is used as the sheath 31.
To the distal end of the sheath 31, there is attached a cover member 32 formed of metal or the like.
The cover member 32 covers the proximal ends of the forceps pieces 11, 12, the link members 15, 16, and the connecting member 17. In the cover member 32, the forceps rotating shaft 13 is fixed. As a result, the forceps rotating shaft 13 is fixed so as not to move with respect to the sheath portion 30.

In a state with the operation member 20 being moved (retracted) farthest to the proximal end side, the connecting member 17 is present outside the sheath portion 30. Namely, the operation member connecting portion 17B, which is a connecting portion between the operation member 20 and the connecting member 17, is set so as not to go into the sheath 31 even if the operation member 20 is retracted to its limit.
In a state with the operation member 20 being moved (advanced) farthest to the distal end side, only the hard portion 20A is exposed to the distal end side of the sheath 31. Namely, an uncoated normal portion 20B does not protrude forward farther than the distal end of the sheath 31, and is not exposed to the outside of the sheath portion 30.
The size of the substantially-cylindrical operation member connecting portion 17B in the shaft line direction, the length of the operation member 20, and the size of the hard portion 20A in the axis line direction of the operation member 20 are set so as to satisfy the aforementioned two conditions.

The motion in use of the treatment tool 1 constructed as described above will be described for the case where it is attached to one of the aforementioned slave arms 200a to 200d.
Firstly, the operator Op attaches the treatment tool 1 to a desired slave arm, and connects the proximal end of the operation member 20 to the adaptor of the slave arm. The cover member 32 is fixed to a base body (not shown in the figure) which is a part of the slave arm. The base body is rotatable about a predetermined base body rotating axis (treatment portion rotating axis). The rotation of the base body causes the cover member 32 to rotate about the base body rotating axis. In addition, the treatment portion 10 is moved to swing. Thereby, it is possible to change the direction of the gripping portion 14.
The operation member, such as a wire, for rotating the base body is connected to a motive power unit such as a motor provided in the slave arm. Namely, the slave arm is provided with a treatment portion rotating mechanism including the base body and the motive power unit.

When the operator Op performs a predetermined operation on a corresponding master arm, the motive power unit of the slave arm is driven via a slave control circuit 400a. The motive power generated in the motive power unit is converted to a linear motion via an adaptor. By the linear motion, the operation member 20 is advanced and retracted.

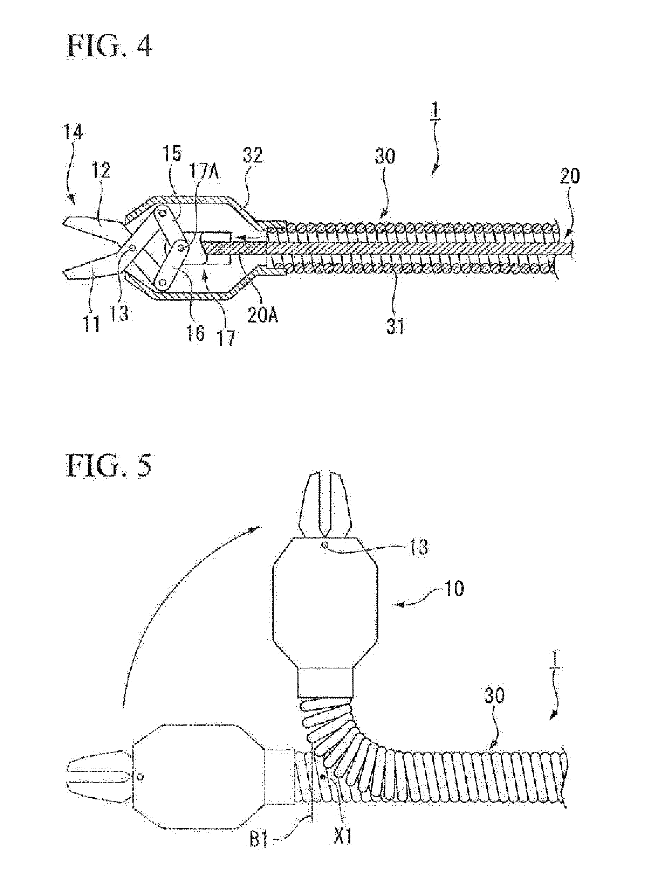

When the operation member 20 is advanced, the connecting member 17 connected to the operation member 20 is advanced with respect to the sheath portion 30. However, the forceps rotating shaft 13 is fixed to the cover member 32, and hence, is not advanced with respect to the sheath portion 30. Therefore, the connecting rotating shaft 17A is brought closer to the forceps rotating shaft 13, causing the link members 15, 16 to rotate with respect to the forceps pieces 11, 12, and the connecting member 17. As a result, the first forceps piece 11 and the second forceps piece 12 are rotated about the forceps rotating shaft 13. This opens the gripping portion 14 as shown in FIG. 4.
When the operation member 20 is retracted, the connecting rotating shaft 17A is spaced away from the forceps rotating shaft 13. This closes the gripping portion 14 through the motion reverse to that described above. Therefore, with the operation member 20 being advanced and retracted, the gripping portion 14 is opened and closed. As a result, it is possible to perform a desired manipulation, for example, grasping a target tissue or grasping a member necessary for a treatment such as a curved needle and a suture thread.

To change the direction of the gripping portion 14, the base body is rotated by the motive power unit via the slave arm. Then, the cover member 32 fixed to the base body rotates about the base body rotating axis, leading to a swing motion of the treatment portion 10.
FIG. 5 shows a state in which the swing motion of the treatment portion 10 is performed to its limit. In the present embodiment, a base body rotating axis X1 is parallel to the shaft line of the forceps rotating shaft 13 when the treatment tool 1 is attached to the slave arm. In addition, the base body rotating axis X1 is located at a position at which the sheath portion 30 in a straight line crosses the central axis line of the sheath portion 30. In a state with the sheath portion 30 being in a straight line and the operation member 20 being retracted as much as possible, the length of the hard portion 20A is set so that the boundary B1 between the hard portion 20A and the normal portion 20B of the operation member 20 is located on a side farther forward than the base body rotating axis X1, namely, is closer to the treatment portion 10 than the base body rotating axis X1 is.

As described above, according to the treatment tool 1 of the present embodiment, also in a state where the operation member 20 is retracted as much as possible, the operation member connecting portion 17B as a connecting portion between the treatment portion 10 and the operation member 20 is set to a position that does not go into the internal portion of the sheath portion 30. Therefore, it is possible to set an inner diameter of the sheath portion 30 in consideration only of the diameter of the operation member 20. As a result, it is possible to make the diameter of the treatment tool 1 smaller. It is also possible to make the difference between the inner diameter of the sheath portion 30 and the outer diameter of the operation member 20 smaller to decrease the gap there between, thus favorably preventing the operation member 20 from buckling in the sheath portion 30.

Furthermore, in a state where the operation member 20 is advanced as much as possible, the length of the hard portion 20A is set so that only the hard portion 20A is positioned outside the sheath portion 30. Therefore, when the pair of forceps pieces 11, 12 of the treatment portion 10 is opened, it is possible to favorably prevent the operation member 20 from buckling outside the sheath portion 30, enabling the operation to be securely transmitted to the treatment portion 10.

In addition, the hard portion 20A is formed by a part of the operation member 20 being subjected to coating. As a result, the treatment tool is easy to manufacture because its hard portion is formed with ease.

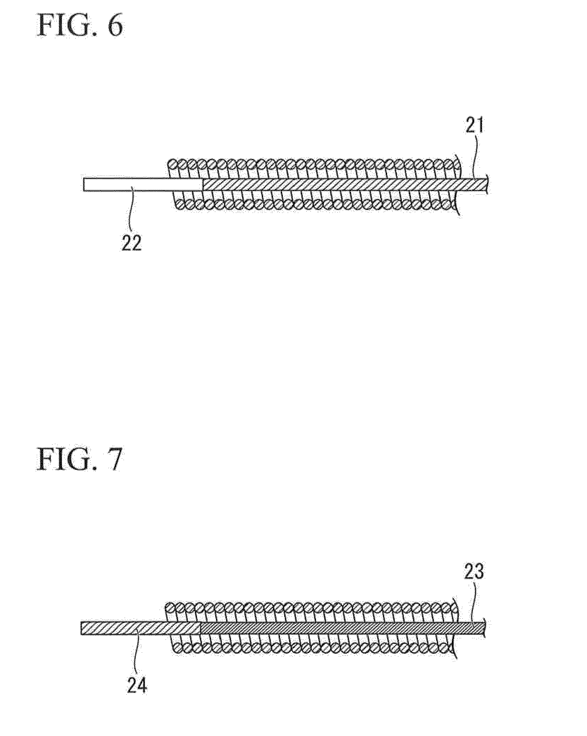

In the present embodiment, the description has been for the case where coating is used for forming a hard portion in a part of the operation member by way of example. However, the method of forming a hard portion is not limited to this.
For example, as is the case with a modification shown in FIG. 6, to one end of a stranded wire 21 that is used as the normal portion of the operation member, a single-stranded wire 22, a rod, or the like with higher rigidity may be connected by welding or the like, to thereby form a hard portion.
Furthermore, as is the case with a modification shown in FIG. 7, to one end of a stranded wire 23 that is used as the normal portion, a stranded wire 24 made of strands thicker than those of the stranded wire 23 may be connected by welding or the like, to thereby form a hard portion.

While one embodiment of the present invention has been described, the technical scope of the present invention is not limited to the embodiment. A variety of modifications can be made to the constituent elements or any of the constituent elements can be deleted without departing from the spirit or scope of the present invention.

For example, in the aforementioned embodiment, the description has been for the case of the treatment tool to be connected to the manipulator. However, other than this, the invention may be used as, for example, a treatment tool for an endoscope that is used by being inserted through a forceps channel of the endoscope. In this case, a known operation portion including: an operation portion main body; and a slider that is slidably attached to the operation portion main body may be provided. Then, the operation portion main body may be connected to a proximal end of a sheath portion, and a proximal end of an operation member may be connected to the slider.

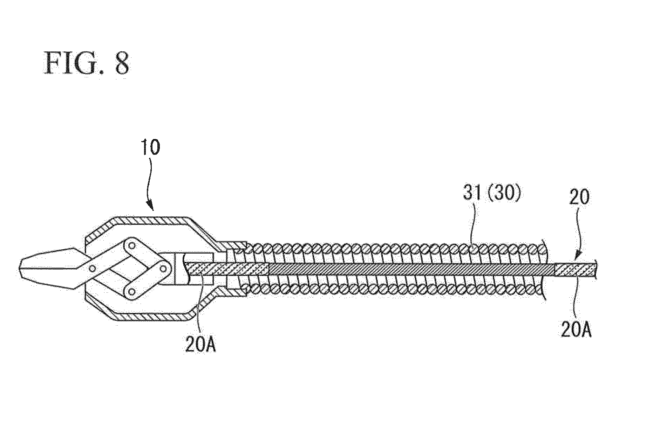

In the operation member 20 of the treatment tool according to the present embodiment, a hard portion 20A may be provided also to the opposite end to the end to which the treatment portion 10 is connected, as is shown in a modification of FIG. 8. As a result, even with the construction in which the proximal end of the operation member 20 is exposed to the outside of the sheath portion 30 in a state with the operation member 20 being retracted as much as possible, it is possible to favorably prevent the occurrence of buckling in the operation member 20 when the operation member 20 is advanced from the state, enabling the operation to be securely transmitted to the treatment portion.

Furthermore, in the treatment tool according to the present embodiment, the treatment portion may not include the link members. In this case, two operation members may be provided, and each operation member provided with a hard portion may be connected to each proximal end of each forceps piece. As a result, advancing and retracting the two operation members can cause the pair of forceps pieces to open/close. At this time, the proximal ends of the two operation members may be stranded or bundled into one integrated body to facilitate the advancing/retracting operation.

Furthermore, in the treatment tool according to the present embodiment, one of the pair of forceps pieces may be fixed so as not to rotate about the forceps rotating shaft. Also in this case, with an operation member with a hard portion being connected to the other forceps piece that is rotatable about the forceps rotating shaft, it is possible to favorably open/close the treatment portion by relatively rotating the pair of forceps pieces about the forceps rotating shaft.

In addition, in the aforementioned embodiment, the description has been for the case where the treatment portion rotating mechanism is provided in a slave arm side that is used as a manipulator. However, the treatment portion rotating mechanism may be provided in a treatment tool side. In this case, the cover member may be formed in a larger size, and the treatment portion rotating axis may be provided at a position that satisfies the aforementioned conditions.

### Industrial Applicability

According to the treatment tool of the present embodiment, the connecting portion in which the treatment portion and the operation member are connected with each other is set to be located a position where the connecting portion is not inserted in the sheath portion even when the operation member is retracted as much as possible. For this reason, the inner diameter of the sheath portion can be set in consideration only of the diameter of the operation member, and therefore the diameter of the medical treatment tool can be easily reduced.

### Brief Description of the Referenced Symbols

- 1:: medical treatment tool
- 10:: treatment portion
- 11:: first forceps piece
- 12:: second forceps piece
- 13:: forceps rotating shaft
- 20:: operation member
- 20A:: hard portion
- 22:: single-stranded wire (hard portion)
- 23:: stranded wire (hard portion)
- 30:: sheath portion
- 200a, 200b, 200c, 200d:: slave arm (manipulator)
- X1:: treatment portion rotating axis

## Claims

1. A medical treatment tool comprising:
a treatment portion having a pair of forceps pieces that are coupled to each other so as to be relatively rotatable about a forceps rotating shaft;
an operation member at least a part of which has flexibility and a distal end of which is connected to a proximal end of the treatment portion; and
a sheath portion which has flexibility and through which the operation member is inserted so as to be capable of advancing and retracting in a longitudinal direction of the sheath portion, wherein
in the operation member, a range with a predetermined length from a distal end is a hard portion with rigidity higher than that of an other portion,
a connecting portion between the treatment portion and the operation member is set so as to be located outside the sheath portion in a state with the operation member being retracted as much as possible, and
a length of the hard portion is set so that the other portion is not exposed to an outside of the sheath portion in a state with the operation member being advanced as much as possible.

2. The medical treatment tool according to claim 1,
wherein the hard portion is formed by subjecting a surface of the operation member to coating.

3. A manipulator comprising the medical treatment tool according to claim 1 or 2.

4. The manipulator according to claim 3, further comprising
a treatment portion rotating mechanism that rotates the treatment portion about a treatment portion rotating axis,
wherein in a state with the sheath portion being in a straight line and the operation member being retracted as much as possible, a boundary between the hard portion and the other portion is closer to the treatment portion than the treatment portion rotating axis.
